# EUROPEAN PATENT APPLICATION

(11) **EP 4 623 820 A1**
(43) Date of publication of application: **01.10.2025**
(21) Application number: 25156949.7
(22) Date of filing: 10.02.2025
(51) Int. Cl.: A61B 5/145, A61B 5/00

(54) **METHOD OF PROVIDING NOTIFICATION OF EVENT**

(30) Priority: 29.03.2024 KR 20240043288 U
(71) Applicant: I-SENS, INC., Seoul 06646 (KR)
(72) Inventor: KU, Ye Sol, Seoul (KR)
(74) Representative: HGF

(57) **Abstract**

An embodiment may provide a method of providing an event notification in a glucose monitoring system, the method including determining whether an event occurs; in response to the occurrence of the event, determining whether an event occurrence time falls within a signal loss section; and outputting a notification corresponding to the event depending on whether the event occurrence time falls within the signal loss section.

## Description

### CROSS-REFERENCE TO RELATED PATENT APPLICATION

The present application claims priority under 35 U.S.C. § 119(a) to Korean patent application number 10-2024-0043288 filed on March 29, 2024, in the Korean Intellectual Property Office, the entire disclosure of which is incorporated by reference herein.

### BACKGROUND OF THE INVENTION

### 1. Field

Embodiments of the present disclosure relate to providing a notification of an event in a glucose monitoring system, and more specifically, to a technology for providing a notification corresponding to an event when a signal loss occurs during output of the notification.

### 2. Description of the Related Art

Recently, with the advancement of medical technology, various medical devices that are attached to a user's body have been developed and sold. A medical device attached to the user's body may be useful for monitoring biometric information or providing treatment by being attached to the skin of a chronic disease patient.

For example, chronic diseases such as diabetes require continuous management, and a medical device that is attached to the skin and measures glucose may be used to monitor glucose in diabetic patients. Diabetes is characterized by almost no noticeable symptoms in the early stages, but as the disease progresses, symptoms characteristic of diabetes appear, such as polydipsia, polyphagia, polyuria, weight loss, general malaise, itchy skin, cases where wounds on the hands and feet do not heal and persist for a long time, and etc. As diabetes progresses further, complications such as vision impairment, high blood pressure, kidney disease, stroke, periodontal disease, muscle spasms, neuralgia, and gangrene appear. In order to diagnose diabetes and manage it to prevent it from developing into complications, systematic glucose measurement and treatment must be carried out together.

For diabetic patients and people who have not developed diabetes but have more sugar than normal detected in their blood, many medical device manufacturers provide various types of glucose meters that may measure glucose.

There are two types of glucose meters, one where the user collects blood from the fingertip and measures glucose on a one-time basis, and one where the user attaches it to the stomach, arm, etc., and continuously measures glucose.

In the case of diabetic patients, they generally go back and forth between hyperglycemia and hypoglycemia, and emergency situations occur during hypoglycemia, and loss of consciousness or prolonged hypoglycemia without sugar supply may result in death. Therefore, immediate detection of hypoglycemia is very important for diabetic patients, but glucose meters that measure glucose intermittently have limitations in accurately detecting this condition.

Recently, to overcome these limitations, a continuous glucose monitoring system (CGMS), which is inserted into the human body and measures glucose levels at intervals of several minutes, has been developed and used. In order to minimize the user's pain and resistance following blood collection, the CGMS may measure glucose continuously after inserting a needle-shaped transdermal sensor into areas where pain is relatively less, such as the stomach or arm.

The CGMS includes a sensor transmitter that is inserted into the user's skin to measure glucose in the body and transmit the measured glucose level, and a terminal that outputs the received glucose level.

The terminal may send various notifications to the user when outputting the glucose levels. Notifications may occur according to conditions related to changes in glucose levels, the usage environment of the sensor transmitter, etc. The user may set the conditions for the notifications to ring or the method of outputting the notifications (for example, sound or vibration, the notification cycle, etc.) through the terminal, and may receive notifications according to the settings.

In addition, the terminal that communicates with the sensor transmitter in the CGMS transmits and receives glucose information wired or wirelessly, and the terminal must continuously receive data packets containing glucose information from the sensor transmitter. However, if the terminal fails to continuously receive glucose information from the sensor transmitter due to a temporary communication interruption between the sensor transmitter and the terminal or the user's inexperienced actions, or if the sensor transmitter and the terminal are separated from each other by a distance that prevents them from communicating with each other for a considerable period of time, the terminal may fail to receive the user's glucose information during that time. In this way, signal loss may occur in which the terminal fails to receive data including glucose information from the sensor transmitter.

However, a situation may occur in which the terminal sounds an alarm while it is unable to receive biometric information from the sensor transmitter due to signal loss. If the terminal does not sound an alarm because it is unable to receive data during signal loss, even though it is hyperglycemia or hypoglycemia before the signal loss or such a state is expected, it may pose a health risk to the user. Accordingly, when events such as high and low glucose values, sudden fluctuations in glucose values, sensor replacement, calibration of glucose values, low battery of a sensor, and connection with a health care manager occur during signal loss, the terminal needs to efficiently process notifications about the events.

### SUMMARY OF THE INVENTION

Against this background, one object of embodiments of the present disclosure is to provide a notification of an event when a signal loss occurs while outputting a notification.

In addition, another object of embodiments of the present disclosure is to provide a notification of an event when a re-notification or snooze is output during a signal loss after a notification due to an event, or when an event occurs during a signal loss and a re-notification or snooze due to the event is output after a signal loss.

In order to achieve the above described objects, one embodiment may provide a method of providing an event notification in a glucose monitoring system, the method including: determining whether an event occurs; in response to the occurrence of the event, determining whether an event occurrence time falls within a signal loss section; and outputting a notification corresponding to the event depending on whether the event occurrence time falls within the signal loss section.

In the method, the outputting of the notification corresponding to the event may include outputting the notification corresponding to the event in the signal loss section.

In the method, the outputting of the notification corresponding to the event may include outputting or stopping output of the notification corresponding to the event during the signal loss section depending on a type of the event, and the event includes high and low glucose values, sudden fluctuations in glucose values, sensor replacement, calibration of glucose values, low battery of a sensor, or connection with a health care manager.

In the method, the outputting of the notification corresponding to the event may include determining to output the notification corresponding to the event if the event has a higher priority than a signal loss.

In the method, the notification may include a re-notification or a snooze.

In the method, the signal loss section may occur during a re-notification or a snooze after the notification corresponding to the event, and the outputting of the notification corresponding to the event may include outputting or stopping output of the re-notification or the snooze during the signal loss section.

In the method, the event may include high and low glucose values, sudden fluctuations in glucose values, sensor replacement, calibration of glucose values, low battery of a sensor, or connection with a health care manager.

In the method, the outputting of the notification corresponding to the event may include determining to output the notification corresponding to the event if the event has a higher priority than a signal loss.

In the method, the event may occur in the signal loss section, and the outputting of the notification corresponding to the event may include outputting or stopping output of a re-notification or a snooze of the notification corresponding to the event after a signal loss ends.

In the method, the event may include high and low glucose values, sudden fluctuations in glucose values, sensor replacement, calibration of glucose values, low battery of a sensor, or connection with a health care manager.

In the method, the outputting of the notification corresponding to the event may include determining to output the re-notification or the snooze if the event has a higher priority than the signal loss.

As described above, according to the embodiments, by determining and controlling whether to output a notification due to an event occurrence during signal loss, it is possible to provide glucose information to a user in a timely manner and prevent health hazards caused by absence of a notification.

### BRIEF DESCRIPTION OF THE DRAWINGS

FIG. 1 is a diagram for schematically explaining a glucose monitoring system according to an embodiment.
FIG. 2 is a diagram for explaining an applicator for attaching a sensor transmitter to a human body according to an embodiment.
FIG. 3 is a diagram for explaining a process of attaching a sensor transmitter to a human body using an applicator according to an embodiment.
FIG. 4 is a configuration diagram of a sensor transmitter according to an embodiment.
FIG. 5 is a configuration diagram of a terminal according to an embodiment.
FIG. 6 is an example diagram in which biometric information is generated in a sensor transmitter or a terminal according to an embodiment.
FIG. 7 an example diagram in which a data packet is generated in a sensor transmitter or a terminal according to an embodiment.
FIG. 8 is a flowchart for explaining a method of transmitting and receiving biometric information between a sensor transmitter and a terminal according to an embodiment.
FIG. 9 is a flowchart for explaining a method of providing a notification of an event by a terminal according to an embodiment.
FIG. 10 is a diagram for explaining a method of providing a notification of an event by a terminal according to an embodiment.
FIG. 11 is a flowchart for explaining a method of determining whether to output a notification and providing a notification of an event by a terminal according to an embodiment.
FIG. 12 is a diagram for explaining notifications provided depending on a type of an event according to an embodiment.
FIG. 13 is a diagram for explaining an example of a method of providing a notification of an event by a terminal according to an embodiment.
FIG. 14 is a flowchart for explaining an example of a method of providing a notification of an event by a terminal according to an embodiment.
FIG. 15 is a diagram for explaining another example of a method of providing a notification of an event by a terminal according to an embodiment.
FIG. 16 is a flowchart for explaining another example of a method of providing a notification of an event by a terminal according to an embodiment.

### DETAILED DESCRIPTION

In describing the present disclosure, if it is determined that related known functions may unnecessarily obscure the gist of the present disclosure as they are obvious to those skilled in the art, detailed descriptions thereof will be omitted.

The terms used in the present application are used merely to describe particular embodiments and are not intended to limit the present disclosure. Singular expressions include plural expressions unless the context clearly indicates otherwise. In the present application, it should be understood that terms such as "comprise", "include", or "have" are intended to designate the presence of features, numbers, steps, operations, components, parts, or combinations thereof described in the specification, and they do not preclude the possibility of the presence or addition of one or more other features, numbers, steps, operations, components, parts, or combinations thereof.

Terms such as first and second are merely identifiers used to distinguish identical or corresponding components, and the identical or corresponding components are not limited by terms such as first, or second.

Hereinafter, embodiments according to the present disclosure will be described in detail with reference to the accompanying drawings, and in describing with reference to the accompanying drawings, identical or corresponding components will be assigned the same drawing numbers and overlapping descriptions thereof will be omitted.

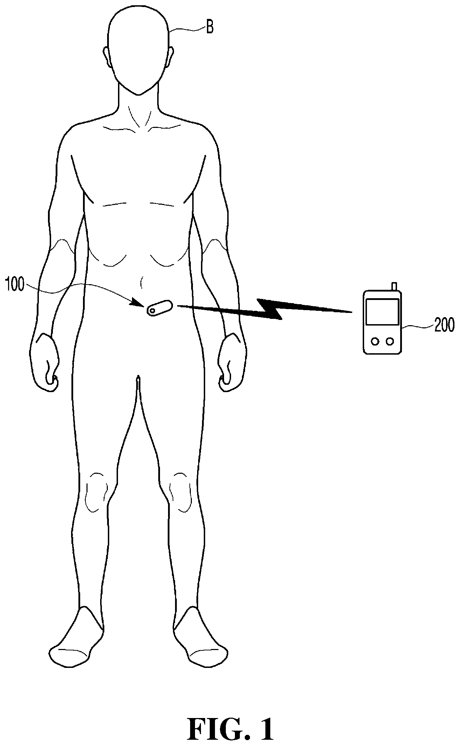

FIG. 1 is a diagram for schematically explaining a glucose monitoring system according to an embodiment.

Referring to FIG. 1, a glucose monitoring system 10 (hereinafter referred to as a "system") according to an embodiment may include a sensor transmitter 100 and a terminal 200.

The sensor transmitter 100 is attached to a human body B, and when the sensor transmitter 100 is attached to the human body B, one end of a sensor of the sensor transmitter 100 is inserted into the skin to periodically extract body fluids from the human body and measure glucose.

The terminal 200 may receive a biosignal including glucose information from the sensor transmitter 100, generate glucose information from the biosignal, and output to the user. The terminal 200 may include, but is not limited to, various devices such as smartphones, mobile phones, tablet PCs, desktops, and laptops, and may have a communication interface capable of communicating with the sensor transmitter 100 and may include a device on which a program or application may be installed.

The sensor transmitter 100 may periodically transmit measured biosignals to the terminal 200 at the request of the terminal 200 or at a set time. For data communication between the sensor transmitter 100 and the terminal 200, the sensor transmitter 100 and the terminal 200 may be connected to each other via a wired connection such as a USB cable or wirelessly using methods such as infrared communication, NFC communication, or Bluetooth.

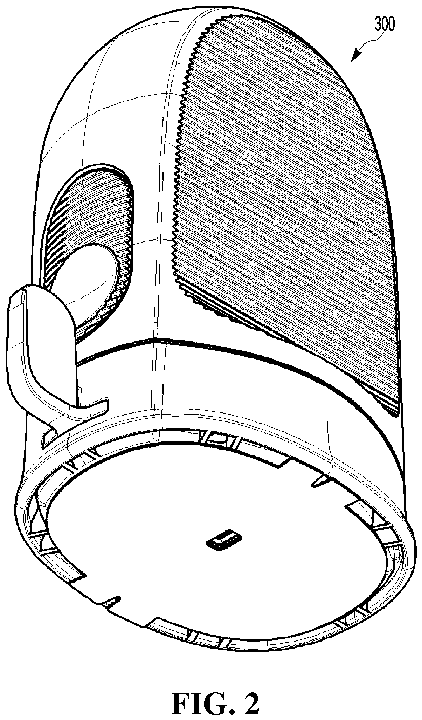

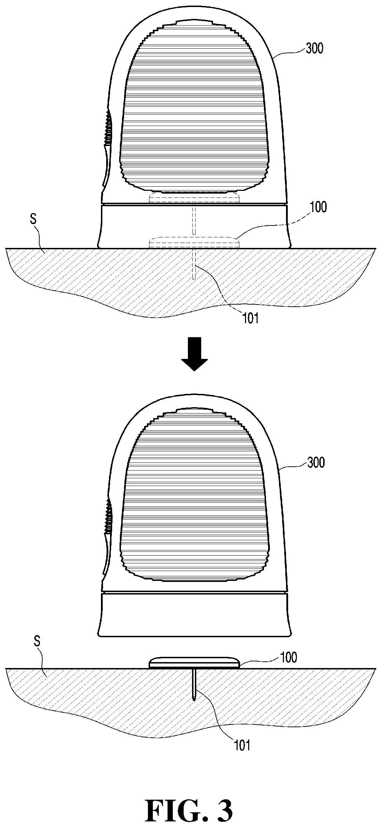

FIG. 2 is a diagram for explaining an applicator for attaching a sensor transmitter to a human body according to an embodiment, and FIG. 3 is a diagram for explaining a process of attaching a sensor transmitter to a human body using an applicator according to an embodiment.

Referring to FIGS. 2 and 3, an applicator 300 according to an embodiment has the sensor transmitter 100 inside and operates to discharge the sensor transmitter 100 to the outside and attach it to a specific body part of the user through manipulation by the user. The applicator 300 is formed in a shape with one side open, and the sensor transmitter 100 is installed in the applicator 300 through the open side of the applicator 300.

When attaching the sensor transmitter 100 to a part of the body using the applicator 300, in order to insert one end of a sensor provided in the sensor transmitter 100 into the skin, the applicator 300 may include a needle (not shown) formed to surround one end of the sensor inside, a first elastic member (not shown) that pushes the needle and one end of the sensor together into the skin, and a second elastic member (not shown) for pulling out only the needle. Through this configuration of the applicator 300, the needle and one end of the sensor may be simultaneously inserted into the skin by decompressing the first elastic member (not shown) disposed in a compressed state inside the applicator 300. When one end of the sensor is inserted into the skin, only the needle is pulled out by decompressing the compressed second elastic member (not shown). The user may safely and easily attach the sensor transmitter 100 to the skin through the applicator 300.

Looking in detail at the process of attaching the applicator 300 to the human body B, with a protective cap (not shown) removed, the open side of the applicator 300 is brought into close contact with the skin S of a specific area of the human body B. When the applicator 300 is operated in this way while the applicator 300 is in close contact with the skin S of the human body B, the sensor transmitter 100 is discharged from the applicator 300 and may be attached to the skin S. Here, one end of the sensor 101 is disposed at a lower part of the sensor transmitter 100, exposed from the sensor transmitter 100, and one end of the sensor 101 may be partially inserted into the skin S through the needle provided in the applicator 300. Therefore, the sensor transmitter 100 may be attached to the skin S with one end of the sensor 101 inserted into the skin S.

Here, an adhesive tape may be provided on the surface of the sensor transmitter 100 in contact with the human body B so that the sensor transmitter 100 may be fixedly attached to the skin S of the human body B. Therefore, when the applicator 300 is separated from the skin S of the human body B, the sensor transmitter 100 may be fixedly attached to the skin S of the human body B by the adhesive tape.

Afterwards, when power is applied to the sensor transmitter 100, the sensor transmitter 100 communicates with the terminal, and the sensor transmitter 100 may transmit biosignals including glucose information to the terminal. The sensor transmitter 100 may generate not only glucose information but also various biometric information, and hereinafter, it will be explained that glucose information is measured as an example of biometric information.

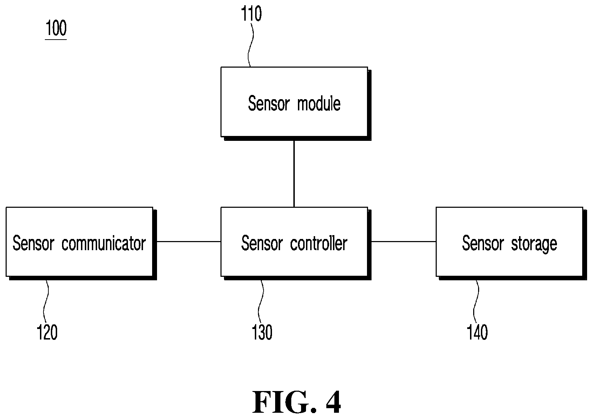

FIG. 4 is a configuration diagram of a sensor transmitter according to an embodiment.

Referring to FIG. 4, the sensor transmitter 100 according to an embodiment may include a sensor module 110, a sensor communicator 120, a sensor controller 130, and a sensor storage 140.

The sensor module 110 may include at least one sensor that is inserted into the human body and senses biomass. At least one sensor may measure biomass and generate biosignals. The biosignal is an analog signal and may include a current value.

The sensor communicator 120 may exchange data or information with the terminal. For example, the sensor communicator 120 may transmit biosignals received from the sensor module 110 or data stored in the sensor storage 140 (for example, biometric information) to the terminal.

The sensor controller 130 may control the overall configuration of the sensor transmitter 100, including the sensor module 110, the sensor storage 140, and the sensor communicator 120. For example, the sensor controller 130 may receive a control signal from the terminal and control the configuration of the sensor transmitter 100 accordingly. In addition, the sensor controller 130 may process biosignals. For example, the sensor controller 130 may convert biosignals into analog or digital form or perform processing to remove noise as needed.

Data or information may be stored in the sensor storage 140. For example, the sensor storage 140 may store data on biomass measured by the sensor module 110, for example, the current value of the biosignal or its digital form data, or data received from the terminal, for example, the command value of the control signal.

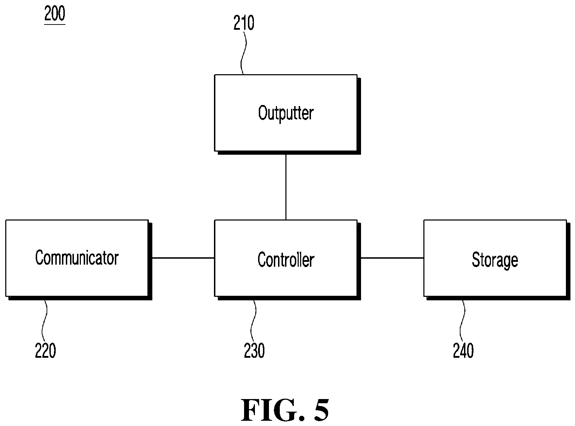

FIG. 5 is a configuration diagram of a terminal according to an embodiment.

Referring to FIG. 5, the terminal 200 according to an embodiment may include an outputter 210, a communicator 220, a controller 230, and a storage 240.

The outputter 210 may output biometric information included in the biosignal, for example, glucose information, so that the user may check it. For example, the outputter 210 may display glucose information as numerical levels (values) or a graph processed from the numerical levels.

The communicator 220 may communicate with the sensor communicator of the sensor transmitter and exchange data or information. For example, the communicator 220 may receive a biosignal containing information (biometric information) about biomass measured by the sensor transmitter. Here, the communicator 220 may receive primarily processed biosignals from the sensor transmitter. Preferably, the processed biosignal may include discrete data (discontinuous data) in which a current value, which is an analog signal, is converted into digital data. If the current value is sampled every cycle, digital discrete data may be generated. Alternatively, the communicator 220 may transmit a control signal for controlling the sensor transmitter to the sensor transmitter.

Data or information may be stored in the storage 240. For example, data received from the sensor transmitter, for example, biometric information, may be stored in the storage 240. Here, biometric information may include digital data representing current values as glucose information. Alternatively, data input from the user or environment setting data for setting the operating environment of the terminal may be stored in the storage 240.

The controller 230 may include at least one processor that executes a program that provides notification of an event in a glucose monitoring system and at least one memory in which the program is stored. The memory and processor included in the controller 230 may be integrated into one chip or may be physically separated.

Memory may be implemented as non-volatile memory devices such as read only memory (ROM), programmable ROM (PROM), erasable programmable ROM (EPROM), electrically erasable programmable ROM (EEPROM), and flash memory, or volatile memory devices such as random access memory (RAM) to store various programs, data, and/or information.

In addition, the controller 230 may generate a glucose value, which is quantified glucose information, from biometric information. To this end, the controller 230 may obtain biometric information in the form of a current value from the sensor transmitter, and preprocess and/or process the current value of the biometric information. The controller 230 may first calculate the sensitivity and generate the glucose value according to this sensitivity.

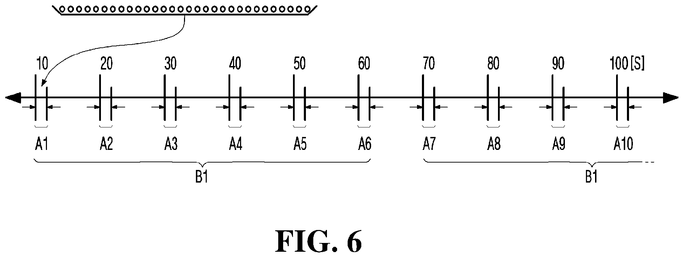

FIG. 6 is an example diagram in which biometric information is generated in a sensor transmitter or a terminal according to an embodiment.

Referring to FIG. 6, biometric information may be generated in a sensor transmitter or a terminal according to an embodiment. Specifically, the sensor transmitter may obtain an analog (continuous) biosignal representing a current value at predetermined intervals, and sample the biosignal to generate digital (discontinuous) data representing the current value. The generated data may be processed in the sensor transmitter or the terminal to generate biometric information. Hereinafter, it is described that biometric information is generated by generating digital data from a biosignal in the sensor transmitter and processing the digital data in the terminal, but is not limited thereto, and part or all of the processing may be performed in the sensor transmitter depending on the embodiment.

For example, the sensor transmitter may obtain a biosignal in an analog form, for example, a current value, measure the biosignal every 10 seconds, and process the measured biosignal to generate a single first data. Specifically, the sensor transmitter may measure (sample) a biosignal 30 times every 10 seconds and generate digital data. The sensor transmitter may remove upper data and lower data from the 30 data, calculate an average value (A1) of the remaining data, and determine this average value (A1) as the single first data. The first data, which is the data of the average value (A1) calculated in this way, is generated in 10 second-units, and as illustrated, six average values (A1 to A6), i.e., six first data, may be generated in 1 minute.

In addition, the sensor transmitter may collect 30 first data for 300 seconds (5 minutes) and transmit them to the terminal. The terminal may receive and process 30 first data from the sensor transmitter every 300 seconds (5 minutes).

The terminal may generate an average value (B1) again using the six first data (average values (A1 to A6)). In generating the average value (B1), the terminal may remove the upper data and lower data among the six average values (A1 to A6) and generate the average value (B1) of the remaining data. The second data, which is the data of the average value (B 1) calculated in this way, is generated in 1 minute-units, and as illustrated, 1 average value (B 1), i.e., 1 second data, may be generated in 1 minute. As described above, in some cases, processing into the second data may also be performed in the sensor transmitter.

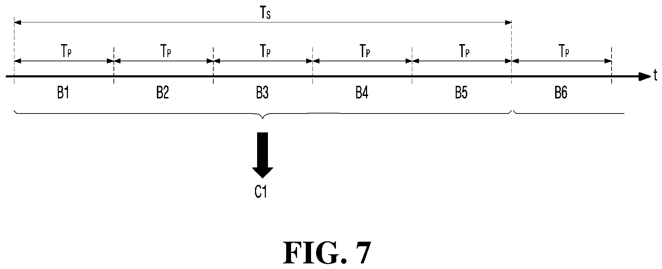

FIG. 7 an example diagram in which a data packet is generated in a sensor transmitter or a terminal according to an embodiment.

Referring to FIG. 7, second data may be processed to generate biometric information in a sensor transmitter or a terminal according to an embodiment. In the above-described example, the terminal may receive 30 first data in 10 second-units from the sensor transmitter and obtain 5 second data (B 1) in 1 minute-units. In addition, the terminal may generate an average value (C1) using the 5 second data (average values (B1 to B5)). In generating the average value (C1), the terminal may remove upper data and lower data among the 5 average values (B1 to B5) and generate an average value (C1) of the remaining data. The third data, which is the data of the average value (C1) calculated in this way, is generated in 5-minute units, and as illustrated, 1 average value (C1), i.e., 1 third data, may be generated in 5 minutes. As described above, in some cases, processing into third data may be performed in the sensor transmitter.

Here, the terminal may sequentially generate second data (B 1 to B5) during a biometric information generation period (Tp) and generate one third data (C1) during a glucose value biometric information generation period (Ts). In the glucose value biometric information generation period (Ts), a glucose value is calculated from the third data (C1) through sensitivity, and in the biometric information generation period (Tp), second data (B 1 to B5) that serve as the basis for the third data (C1) may be generated. In the example described above, the biometric information generation period (Tp) may correspond to 1 minute, and the glucose value biometric information generation period (Ts) may correspond to 5 minutes.

In this way, the third data generated in 5-minute units may undergo a noise removal process by a filter. The filtered third data may be converted into biometric information including a glucose value by applying sensitivity, and such biometric information may be output to the user.

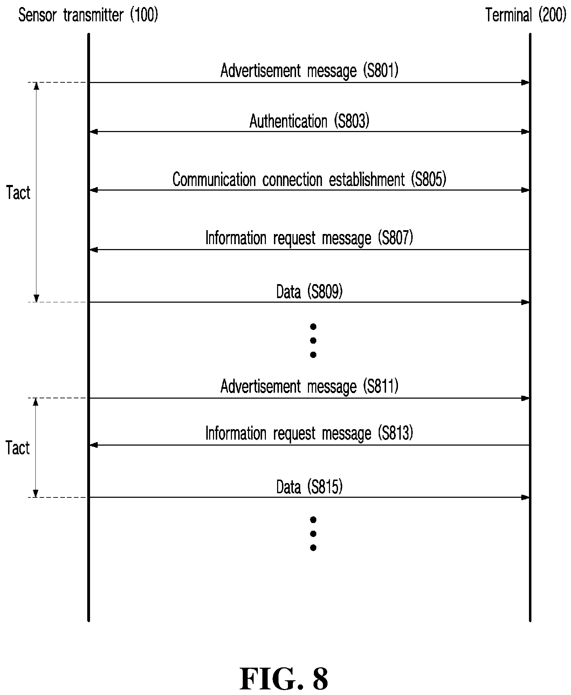

FIG. 8 is a flowchart for explaining a method of transmitting and receiving biometric information between a sensor transmitter and a terminal according to an embodiment.

Referring to FIG. 8, the sensor transmitter 100 and the terminal 200 according to an embodiment may connect communication in order to transmit and receive data including biometric information. Data may be transmitted and received after the communication connection. The sensor transmitter 100 and the terminal 200 may be connected to each other through wired or wireless communication, and may connect communication in a manner such as USB communication, infrared communication, Bluetooth communication, etc.

Specifically, the sensor transmitter 100 and the terminal 200 may transmit and receive differently depending on whether communication is disconnected and a new communication is connected (when connecting initial communication) or only data is transmitted and received after initial communication is connected. First, in a state of communication disconnection, when the sensor transmitter 100 and the terminal 200 establish a new communication connection, the sensor transmitter 100 may transmit an advertisement message to the terminal 200 (step S801). The sensor transmitter 100 may periodically transmit an advertisement message to the terminal 200, and this process may be called advertisement. The terminal 200 may receive the advertisement message and perform authentication with the sensor transmitter 100 (step S803). For example, the sensor transmitter 100 and the terminal 200 may authenticate that they are valid devices through a hash value. Then, the sensor transmitter 100 and the terminal 200 may establish a communication connection (step S805). The communication connection is a state in which data may be transmitted and received immediately without going through a separate initial communication process such as authentication, and the sensor transmitter 100 and the terminal 200 may transmit and receive data in response to an advertisement message at any time while the communication connection is being established. The terminal 200 may transmit an information request message to the sensor transmitter 100 to obtain data including biometric information (for example, 30 first data) (step S807). The sensor transmitter 100 that has received the information request message may transmit data including biometric information (for example, 30 first data) to the terminal 200 in response thereto (step S809).

Once the initial communication is connected, data may be transmitted and received in the communication connection establishment state without a separate authentication process. The sensor transmitter 100 may transmit an advertisement message to the terminal 200 periodically or aperiodically (step S811). The terminal 200 may send an information request message requesting data transmission in response to the advertisement message to the sensor transmitter 100 (step S813). The sensor transmitter 100 may transmit data in response to the information request message (step S815).

Here, the terminal 200 receives data from the sensor transmitter 100 from time to time, but the terminal 200 may receive data in response to the advertisement message only when the sensor transmitter 100 transmits the advertisement message. The sensor transmitter 100 may send this advertisement message to the terminal 200 periodically or aperiodically, and the terminal 200 may request and receive data accordingly. In addition, the sensor transmitter 100 may not continuously send the advertisement message, but may transmit it only in active mode, i.e., while awake. Accordingly, data transmission and reception between the sensor transmitter 100 and the terminal 200 may be performed only during this active mode period (Tact). The sensor transmitter 100 may stand by without sending any data during the inactive mode, i.e, the non-active mode period.

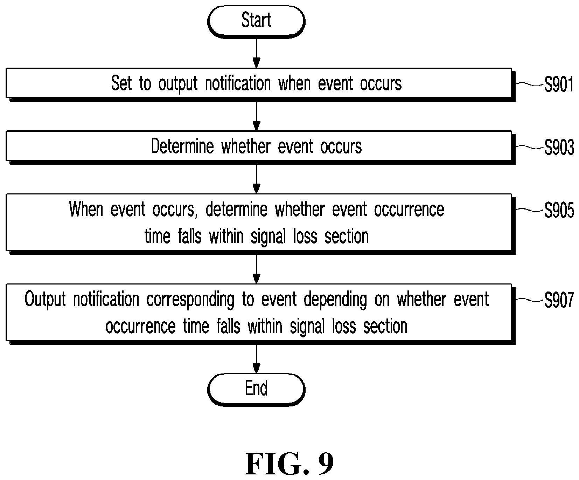

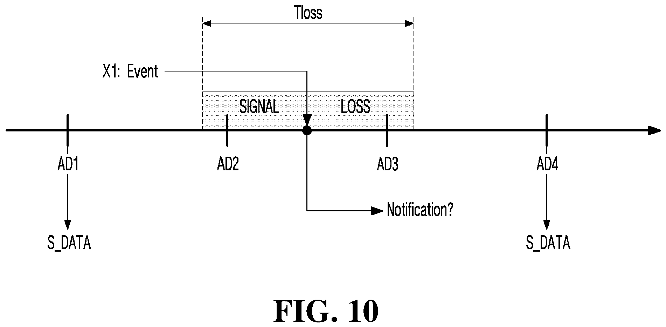

FIG. 9 is a flowchart for explaining a method of providing a notification of an event by a terminal according to an embodiment, and FIG. 10 is a diagram for explaining a method of providing a notification of an event by a terminal according to an embodiment.

Referring to FIG. 9, a flowchart of a method of providing a notification of an event by a terminal according to an embodiment may be illustrated. The terminal may provide various notifications in response to events related to outputting biometric information to a user.

For example, events may include signal loss, high and low glucose values, sudden fluctuations in glucose values, sensor replacement, calibration of glucose values, low battery of a sensor, or connection with a health care manager. Signal loss indicates that the terminal cannot receive biometric information from the sensor transmitter, and if data is not received for a certain period of time (for example, 25 minutes), a notification may be provided to the user. High and low glucose values indicate hyperglycemia and hypoglycemia, and the user may set a notification for high and low glucose values by setting a range (lower and upper limits) that specifies hyperglycemia and hypoglycemia. The sudden fluctuations in glucose values indicate the degree of change (or slope) of glucose value, and if the change is severe, whether it is hyperglycemia or hypoglycemia, a notification may be provided to the user. The sensor replacement indicates that the sensor should be replaced when the sensor reaches the end of its lifespan, and a notification may be provided to the user when the sensor reaches the end of its lifespan. The calibration of glucose values indicates that a calibration value should be input in order to calculate an accurate glucose value, and a notification may be provided to the user when the calibration time comes. The low battery of the sensor may indicate that the battery (power source) of the sensor that is driven by its own battery is exhausted and the sensor should be replaced. A notification may be provided to the user when the remaining battery level is low. The connection with the health manager may indicate that a subject other than the user who takes care of the user's health status is connected by communication with the sensor transmitter through another terminal and receives biometric information from the sensor transmitter. A notification may be provided to the user when the health manager is connected.

Specifically, when the terminal outputs a notification in response to the occurrence of the above event and is in the middle of signal loss, the terminal in the present embodiment may operate as follows.

First, the controller of the terminal may set the outputter to output a notification when an event occurs (step S901). The terminal receives a command for an event and a notification from a user, and the controller of the terminal may control the outputter to output a notification according to the event according to the command.

The controller of the terminal may determine whether an event occurs (step S903). Specifically, the controller of the terminal may detect whether an event occurs according to the above setting. The controller of the terminal may detect whether signal loss, high and low glucose values, sudden fluctuations in glucose values, sensor replacement, calibration of glucose values, low battery of a sensor, and connection with a health care manager occur. For example, the controller of the terminal may determine that signal loss has occurred if data is not received for a certain period of time or longer. Alternatively, the controller of the terminal may determine that hyperglycemia or hypoglycemia has occurred if the glucose value goes out of a boundary specified by the user. Alternatively, the controller of the terminal may determine that an event requiring sensor replacement or glucose value calibration has occurred when the life cycle or calibration cycle of the sensor has arrived. When such an event occurs, the controller of the terminal may control the outputter to output a notification set according to the event.

Normally, the controller of the terminal may determine whether an event occurs, and according to the user's settings, the outputter may output a notification accordingly. In the meantime, there may be cases where signal loss and notification output overlap. For example, while the terminal is in signal loss, the high and low or sudden fluctuations in glucose values may be expected so that the terminal must output a notification for this, or the sensor's lifespan or calibration period may arrive so that the terminal may have to output a notification for this. When such an event occurs, the terminal may output a notification in response to the event. Here, the terminal may determine whether the event occurrence time (hereinafter referred to as 'time X1') is in the signal loss section (step S905). In other words, the terminal may determine whether to output a notification corresponding to the event during the signal loss. The signal loss section may mean the time during which the signal loss continues in the terminal.

Referring to FIG. 10, a situation in which a terminal according to an embodiment outputs a notification when a signal loss occurs may be illustrated.

Specifically, the terminal may receive biometric information (S_DATA) from the sensor transmitter in response to an advertisement message sent by the sensor transmitter at an advertisement timing (AD1) in normal times.

A signal loss may occur at a certain point during communication between the sensor transmitter and the terminal, and the terminal may enter a signal loss section (Tloss). During the signal loss, the terminal may not receive any data, including biometric information, from the sensor transmitter. Unlike the advertisement timing (AD1), the terminal may not receive biometric information at advertisement timings (AD2, AD3) that belong to the signal loss section (Tloss). While the terminal is in a state of signal loss, an event occurs at time X1, and the terminal may have to output a notification corresponding to the event. In this situation, the terminal may determine whether to output a notification. Depending on the determination, the terminal may output a notification or stop outputting the same.

Thereafter, the signal loss is resolved, and the terminal may receive data from the sensor transmitter. The terminal may receive biometric information from the sensor transmitter as usual at the subsequent advertisement timing (for example, the advertisement timing (AD4)).

Here, the advertisement timings (AD1, AD2, AD3, AD4) may be periodic or aperiodic. When the advertisement timings (AD1, AD2, AD3, AD4) are periodic, the advertisement timings (AD1, AD2, AD3, AD4) may be formed at intervals of 5 minutes (300 seconds). As in the example of the data packet described above, in principle, the sensor transmitter may send 30 of collected data to the terminal at once at each advertisement timing (AD1, AD2, AD3, AD4). The advertisement timings (AD1, AD2, AD3, AD4) are repeated, and other advertisement timings may also be formed at intervals of 5 minutes thereafter. However, in the advertisement timings (AD2, AD3) in the signal loss state, the terminal may not receive data. While the biometric information is not received, the terminal may output a blank or gap where the glucose value is not displayed on the user interface.

Referring again to FIG. 9, the controller of the terminal according to an embodiment may determine whether to output a notification for an event that occurs during the signal loss. In other words, the controller of the terminal may output a notification for the event depending on whether the event occurrence time falls within the signal loss section (step S907). In normal times when there is no signal loss, the terminal may output a notification for the event. On the other hand, during the signal loss, the terminal may output a notification for the event, but may not output it depending on the criteria. Here, the notification for the event may include a re-notification and a snooze. Specifically, the controller of the terminal may determine whether to output a notification based on the priority between the event and the signal loss. For example, the controller of the terminal may control the outputter to output a notification during signal loss if the priority of the event is higher than the priority of signal loss. In addition, the controller of the terminal may determine whether to output a notification depending on the type of event. This will be described in detail in the drawings later.

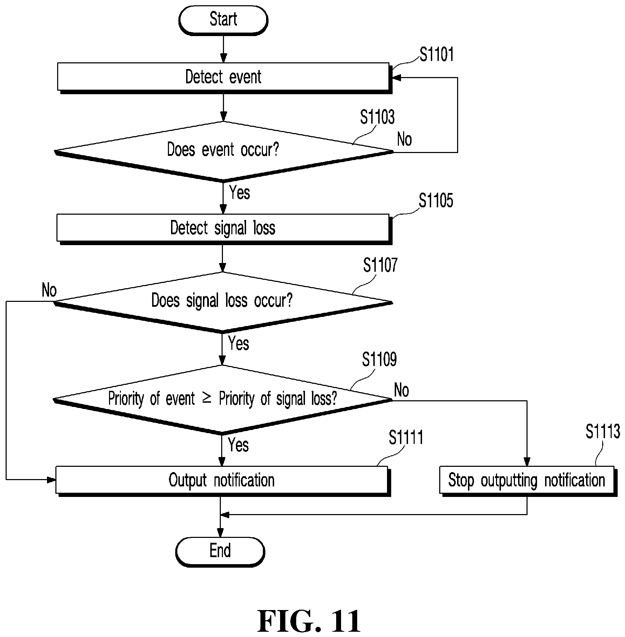

FIG. 11 is a flowchart for explaining a method of determining whether to output a notification and providing a notification of an event by a terminal according to an embodiment.

Referring to FIG. 11, an operation of a terminal to provide a notification during signal loss according to an embodiment may be illustrated.

The terminal may detect and determine whether an event occurs (step S 1101 and step S1103). The terminal may detect and determine whether an event according to a corresponding type (signal loss, high and low glucose values, sudden fluctuations in glucose values, sensor replacement, calibration of glucose values, low battery of a sensor, connection with a health care manager, and the like) occurs.

If it is determined that an event does not occur, the terminal may continuously detect the event (NO of step S1103 and step S1101). If it is determined that an event occurs, the terminal may additionally detect a signal loss (YES of step S 1103 and step S1105). The terminal may determine whether it is currently in a signal loss state.

If it is determined that signal loss does not occur, the terminal may output a notification for the event (NO of step S1107 and step S1111). If it is determined that signal loss occurs, the terminal may compare and determine the priority between the event and the signal loss (YES of step S1107 and step S 1 109). If it is determined that the priority of the event is higher than or equal to the priority of the signal loss, the terminal may output a notification for the event (YES of step S1109 and step S1111). For example, if an event of high and low glucose values, sudden fluctuations in glucose values, or calibration has a higher priority than signal loss, the terminal may output a notification therefor.

Conversely, if it is determined that the priority of the event is lower than the priority of the signal loss, the terminal may not output a notification for the event or may stop outputting the same even if it outputs it (NO of step S1109 and step S1113). For example, if an event of high or low blood glucose values or sudden fluctuations in glucose values has a lower priority than a signal loss, the terminal may not output a notification therefor.

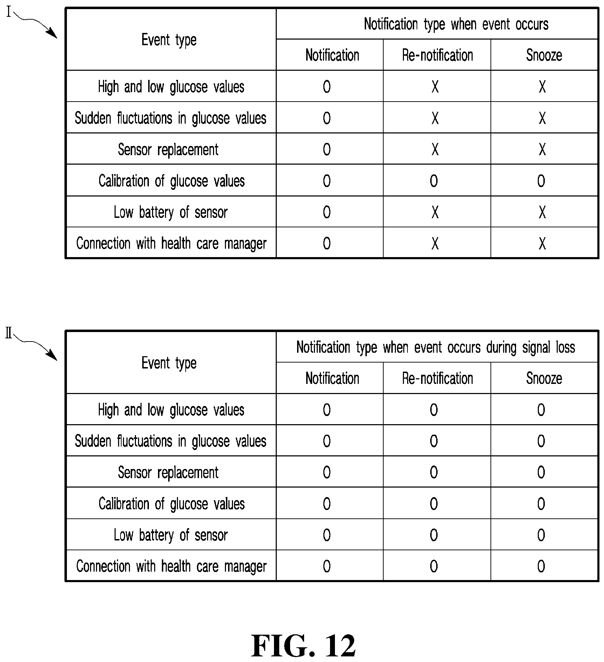

FIG. 12 is a diagram for explaining notifications provided depending on a type of an event according to an embodiment.

Referring to FIG. 12, a notification provided by a terminal according to an embodiment depending on a type of an event may be illustrated. The terminal may determine the type of notification output during normal times or signal loss and the corresponding event differently. Here, the types of notification may include a normal notification, a re-notification, and a snooze. In general, a notification may mean a notification that is initially sounded when an event occurs. A re-notification may mean a notification that is periodically or aperiodically notified again after the initial notification. A snooze may mean a notification that is provided later after a notification is temporarily ignored. Compared to a re-notification, a snooze may have a difference in that it requires a user's operation for the initial notification and the time interval after the initial notification is relatively short.

For example, in I of the present drawing, the types of notifications for normal events are illustrated. In normal times when there is no signal loss, the terminal may provide notifications for high and low glucose values, sudden fluctuations in glucose values, sensor replacement, calibration of glucose values, low battery of a sensor, and connection with a health care manager. However, the terminal may provide re-notification and snooze only for the event of calibration of glucose values. The user may manipulate the terminal to provide re-notification and snooze for other events as well.

On the other hand, in II of the present drawing, the types of notifications for events that occur during signal loss are illustrated. In the case where signal loss occurs and an event occurs during the signal loss, the terminal may identically provide notifications for high and low glucose values, sudden fluctuations in glucose values, sensor replacement, calibration of glucose values, low battery of a sensor, and connection with a health care manager. However, the terminal may provide re-notification and snooze not only for the calibration of glucose values but also for events such as high and low glucose values, sudden fluctuations in glucose values, sensor replacement, low battery of a sensor, and connection with a health care manager. The terminal may determine that the priority of the event is higher than the priority of signal loss according to the above setting. The terminal may output a notification for the event even during signal loss through priority comparison. The user may manipulate the terminal so that these settings may be changed.

Hereinafter, examples of the terminal outputting a notification during signal loss will be described.

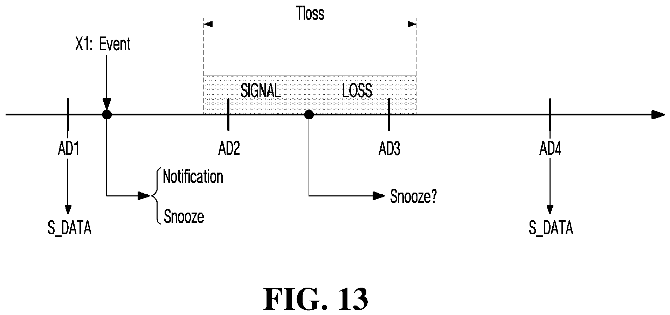

FIG. 13 is a diagram for explaining an example of a method of providing a notification of an event by a terminal according to an embodiment.

Referring to FIG. 13, an example in which a terminal according to an embodiment outputs a notification when a signal loss occurs may be illustrated. In this example, a signal loss occurs after the terminal outputs a notification due to an event, and the terminal may need to provide a notification such as a re-notification or a snooze during the signal loss. When a signal loss occurs while outputting a notification, the terminal determines whether to output the notification, and the terminal may or may not output the notification based on this determination.

Specifically, the terminal may receive biometric information (S _DATA) from the sensor transmitter in response to an advertisement message sent by the sensor transmitter at the advertisement timing (AD1) during normal times.

An event occurs at time X1 while the sensor transmitter and the terminal are communicating, and the terminal may output a notification or a snooze. Snooze is set to ON, and signal loss may occur. The terminal may enter a signal loss section (Tloss) and the time may come to output snooze. In this situation, the terminal may determine whether to output snooze. Depending on the determination, the terminal may output snooze or stop outputting the same.

Here, the terminal may determine whether to output snooze based on the priority between the event and the signal loss. For example, the terminal may output snooze during the signal loss if the priority of the event is higher than the priority of the signal loss. This will be described in detail in the drawings later.

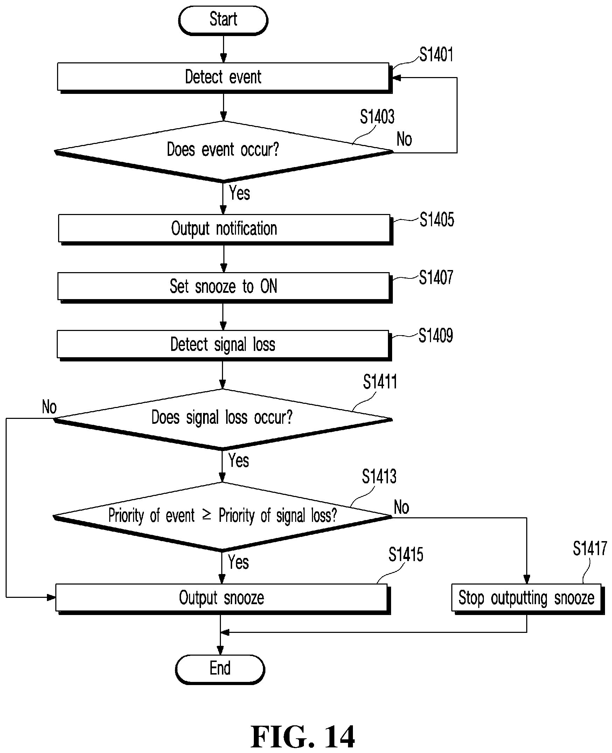

FIG. 14 is a flowchart for explaining an example of a method of providing a notification of an event by a terminal according to an embodiment.

Referring to FIG. 14, an operation of a terminal to provide a notification during a signal loss when a re-notification or a snooze and a signal loss overlap after a notification due to an event according to an embodiment may be illustrated.

The terminal may detect and determine whether an event occurs (step S1401 and step S1403). The terminal may detect and determine whether an event according to the type (signal loss, high and low glucose values, sudden fluctuations in glucose values, sensor replacement, calibration of glucose values, low battery of a sensor, connection with a health care manager, and the like) occurs.

If it is determined that an event does not occur, the terminal may continuously detect the event (NO of step S1403 and step S1401). If it is determined that an event occurs, the terminal may output a notification for the event (YES in step S1403 and step S1405).

The terminal may set the snooze to ON so that the snooze sounds (step S1407). The user may change the settings for the notification by operating the terminal, and may also set whether to output the snooze. The terminal may set the snooze to ON according to the settings of the notification input by the user.

The terminal may stop notifications and continue to receive biometric information after reserving a snooze. During the reception of biometric information, the terminal may detect signal loss and determine whether signal loss occurs (step S1409 and step S1411). The terminal may determine whether it is currently in a signal loss state.

If it is determined that signal loss does not occur, the terminal may output a snooze for the event (NO of step S1411 and step S1415). If it is determined that signal loss occurs, the terminal may compare and determine the priority between the event and the signal loss (YES of step S1411 and step S1413). If it is determined that the priority of the event is higher than or equal to the priority of the signal loss, the terminal may output a snooze for the event (YES of step S1413 and step S1415). For example, if an event of high and low glucose values, sudden fluctuations in glucose values, or calibration has a higher priority than signal loss, the terminal may output a snooze therefor.

Conversely, if it is determined that the priority of the event is lower than the priority of the signal loss, the terminal may not output a snooze for the event or may stop outputting the same even if it outputs it (NO of step S1413 and step S1417). For example, if an event of high or low blood glucose values or sudden fluctuations in glucose values has a lower priority than a signal loss, the terminal may not output a snooze therefor.

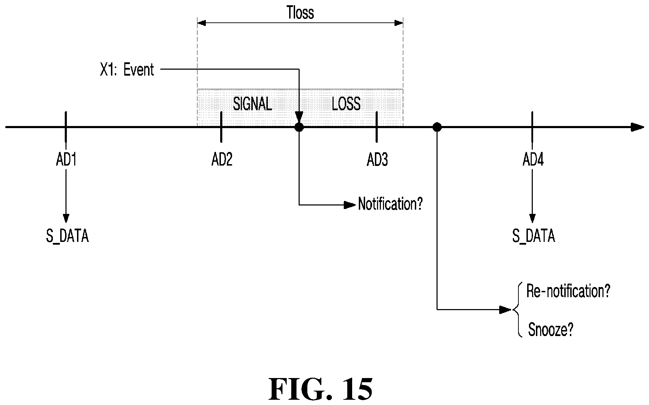

FIG. 15 is a diagram for explaining another example of a method of providing a notification of an event by a terminal according to an embodiment.

Referring to FIG. 15, another example in which a terminal according to an embodiment outputs a notification when a signal loss occurs may be illustrated. In this example, an event occurs during a signal loss, and after the signal loss, the terminal may need to provide a re-notification or snooze due to the event. The terminal determines whether to output a notification for the event that occurs during signal loss, and may or may not output the notification based on the determination. In addition, after the signal loss ends, the terminal determines whether to output a re-notification or snooze for the event, and may or may not output the re-notification or snooze based on the determination.

Specifically, the terminal may receive biometric information (S _DATA) from the sensor transmitter in response to an advertisement message sent by the sensor transmitter at the advertisement timing (AD1) during normal times.

An event occurs at time X1 while the sensor transmitter and the terminal are communicating, and the terminal may determine whether to output a notification. The terminal may output the notification or stop outputting the same based on the determination. This notification becomes the first notification for the event at time X1, and the terminal may output a re-notification or snooze based on a user's input after the signal loss ends. Here, the terminal may determine whether to output the notification based on the priority between the event and the signal loss. For example, the terminal may output the notification during the signal loss if the priority of the event is higher than the priority of the signal loss.

When the signal loss ends, a re-notification or snooze time may come. The terminal may determine whether to output a re-notification or snooze. Depending on the determination, the terminal may output a re-notification or snooze or stop outputting the same. In particular, even if the notification does not ring during the signal loss, it is necessary to determine whether the terminal should output a re-notification or snooze based on a notification that does not ring after the signal loss ends. As during the signal loss, the terminal may determine whether to output a re-notification or snooze based on the priority between the event and the signal loss. For example, the terminal may output a re-notification or snooze after the signal loss ends if the priority of the event is higher than the priority of the signal loss. This will be described in detail in the drawings later.

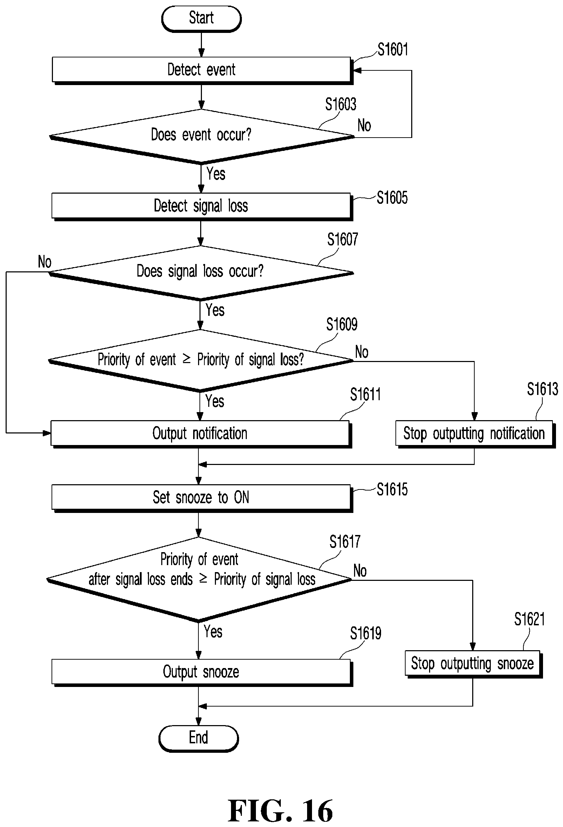

FIG. 16 is a flowchart for explaining another example of a method of providing a notification of an event by a terminal according to an embodiment.

Referring to FIG. 16, an event may occur during a signal loss and an operation of a terminal for providing a re-notification or snooze due to the event after the signal loss according to an embodiment may be illustrated. Here, the terminal providing a snooze will be described as an example.

The terminal may detect and determine whether an event occurs (step S 1601 and step S 1603). The terminal may detect and determine whether an event according to the type (high and low glucose values, sudden fluctuations in glucose values, sensor replacement, calibration of glucose values, low battery of a sensor, connection with a health care manager, and the like) occurs.

If it is determined that an event does not occur, the terminal may continuously detect the event (NO of step S1603 and step S1601). If it is determined that an event occurs, the terminal may additionally detect a signal loss (YES of step S 1603 and step S 1605). The terminal may determine whether it is currently in a signal loss state.

If it is determined that signal loss does not occur, the terminal may output a notification for the event (NO of step S 1607 and step S 1611). If it is determined that signal loss occurs, the terminal may compare and determine the priority between the event and the signal loss (YES of step S 1607 and step S1609). If it is determined that the priority of the event is higher than or equal to the priority of the signal loss, the terminal may output a notification for the event (YES of step S1609 and step S1611). For example, if an event of high and low glucose values, sudden fluctuations in glucose values, or calibration has a higher priority than the signal loss, the terminal may output a notification for this.

Conversely, if it is determined that the priority of the event is lower than the priority of the signal loss, the terminal may not output a notification for the event or may stop outputting the same even if it outputs it (NO of step S1609 and step S1613). For example, if an event of high or low blood glucose values or sudden fluctuations in glucose values has a lower priority than a signal loss, the terminal may not output a notification therefor.

The terminal may set the snooze to ON so that the snooze sounds (step S1615). The user may change the settings for the notification by manipulating the terminal and may also set whether to output the snooze. The terminal may set the snooze to ON according to the settings of the notification input by the user.

The terminal may stop the notification, reserve a snooze, and continue to receive biometric information. While the terminal is receiving biometric information, the signal loss may end and the snooze time may arrive. The terminal may compare and determine the priority between the event and the signal loss (step S1617). If it is determined that the priority of the event is higher than or equal to the priority of the signal loss, the terminal may output a snooze for the event (YES of step S1617 and step S1619). For example, if an event of high and low glucose values, sudden fluctuations in glucose values, or calibration has a higher priority than the signal loss, the terminal may output a snooze for this.

Conversely, if it is determined that the priority of the event is lower than the priority of the signal loss, the terminal may not output a snooze for the event or may stop outputting the same even if it outputs it (NO of step S1617 and step S1621). For example, if an event of high or low blood glucose values or sudden fluctuations in glucose values has a lower priority than a signal loss, the terminal may not output a snooze therefor.

The aspects of the subject matter described herein may be described in the context of computer-executable instructions, such as program modules, that are executed on a computer. Generally, program modules include routines, programs, objects, components, data structures, etc., which perform specific tasks or specific abstract data types.

Alternatively or additionally, the functionality described herein may be performed, at least in part, by one or more hardware logic components. By way of example, and not limitation, exemplary types of hardware logic components that may be used include field-programmable gate array (FPGA), program-specific integrated circuit (ASIC), application-specific standard product (ASSP), system-on-a-chip system (SOC), complex programmable logic device (CPLD), etc.

In addition, the disclosed embodiments may be implemented in the form of a non-transitory recording medium that stores programs and/or instructions executable by a computer. Instructions may be stored in the form of program code, and when executed by a processor, may create program modules to perform operations of the disclosed embodiments. The non-transitory recording medium may be implemented as a non-transitory computer-readable recording medium.

Computer-readable recording media include all types of recording media storing instructions that may be decoded by a computer. For example, there may be read only memory (ROM), random access memory (RAM), magnetic tape, magnetic disk, flash memory, optical data storage, etc.

Although embodiments of the present disclosure have been described above, those skilled in the art will be able to make various modifications and changes to the present disclosure by adding, changing or deleting components, without departing from the spirit of the present disclosure as set forth in the appended claims, which are included within the scope of rights of the present disclosure.

## Claims

1. A method of providing an event notification in a glucose monitoring system, the method comprising:
determining (S903) whether an event occurs;
in response to the occurrence of the event, determining (S905) whether an event occurrence time falls within a signal loss section; and
outputting (S907) a notification corresponding to the event depending on whether the event occurrence time falls within the signal loss section.

2. The method according to claim 1, wherein the outputting (S907) of the notification corresponding to the event comprises outputting the notification corresponding to the event in the signal loss section.

3. The method according to claim 1, wherein the outputting (S907) of the notification corresponding to the event comprises outputting or stopping output of the notification corresponding to the event during the signal loss section depending on a type of the event, and
the event comprises high and low glucose values, sudden fluctuations in glucose values, sensor replacement, calibration of glucose values, low battery of a sensor, or connection with a health care manager.

4. The method according to claim 1, wherein the outputting (S907) of the notification corresponding to the event comprises determining to output the notification corresponding to the event if the event has a higher priority than a signal loss.

5. The method according to claim 1, wherein the notification comprises a re-notification or a snooze.

6. The method according to claim 1, wherein the signal loss section occurs during a re-notification or a snooze after the notification corresponding to the event, and
the outputting (S907) of the notification corresponding to the event comprises outputting or stopping output of the re-notification or the snooze during the signal loss section.

7. The method according to claim 6, wherein the event comprises high and low glucose values, sudden fluctuations in glucose values, sensor replacement, calibration of glucose values, low battery of a sensor, or connection with a health care manager.

8. The method according to claim 6, wherein the outputting (S907) of the notification corresponding to the event comprises determining to output the notification corresponding to the event if the event has a higher priority than a signal loss.

9. The method according to claim 1, wherein the event occurs in the signal loss section, and
the outputting (S907) of the notification corresponding to the event comprises outputting or stopping output of a re-notification or a snooze of the notification corresponding to the event after a signal loss ends.

10. The method according to claim 9, wherein the event comprises high and low glucose values, sudden fluctuations in glucose values, sensor replacement, calibration of glucose values, low battery of a sensor, or connection with a health care manager.

11. The method according to claim 9, wherein the outputting (S907) of the notification corresponding to the event comprises determining to output the re-notification or the snooze if the event has a higher priority than the signal loss.
